# EUROPEAN PATENT SPECIFICATION

(11) **EP 1 272 240 B1**
(45) Date of publication and mention of the grant of the patent: **30.04.2014**
(21) Application number: 01911998.1
(22) Date of filing: 20.03.2001
(51) Int. Cl.: A61M 5/50, A61M 5/31, A61M 5/32

(54) **HYPODERMIC SYRINGE**
HYPODERMATISCHE SPRITZE
SERINGUE HYPODERMIQUE

(30) Priority: 24.03.2000 GB 0007269
(43) Date of publication of application: 08.01.2003
(73) Proprietor: C-Major Limited, Hughenden Vally, Buckinghamshire HP11 4PA (GB)
(72) Inventor: Parker, David William, Greenmount Bury Lancashire BL8 4QQ (GB); Burgess, Colin Hamilton, Ramsbottom lancashire BL0 9QR (GB)
(74) Representative: Boult Wade Tennant
(86) International application number: PCT/GB2001/001228
(87) International publication number: WO 2001/072362

(56) References cited:
- WO-A-94/07553
- WO-A1-01/49347
- WO-A2-2004/030732
- FR-A- 2 739 563
- FR-A- 2 739 564
- US-A- 5 695 472
- US-A- 5 779 677

## Description

### Field of Invention

This invention relates to hypodermic syringes with a retractable needle.

### Background

Over recent years, the use of disposable syringes and needles has become increasingly dangerous. Although the risk of an accidental scratch or puncture by a used needle, known as a needle stick injury, has always existed, the increased risk of infection with, for example HIV or hepatitis, has become a growing concern to those involved in the use of disposable hypodermic syringes.

It is estimated that in the USA, there are approximately 1,000,000 needle stick injuries annually which result in some 20,000 incidences of infection with HIV or hepatitis. These cause a considerable loss of life and the consequent cost is estimated at US$3 billion per annum currently.

With a view to overcoming this difficulty, a number of designs of hypodermic syringes with retractable needles have been proposed. One such design is known from US-A-5 213 628 which discloses a hypodermic syringe including a housing, a plunger, a needle carrier with a needle mounted thereto positionable within the housing with the needle extending therefrom, a sheath mounted to the needle carrier and surrounding the needle, and a spring, the plunger and the spring configured in such a way that when the syringe is used and the plunger reaches the needle carrier it becomes attached thereto and stored energy in the spring is released to retract the needle carrier into the housing.

Similar hypodermic syringes having an arrangement for retracting the needle into the plunger after use are known from US-A-5 324 265, EP-A-0 505 300 and WO91/03269A.

FR 2739564 describes a syringe with a barrel for containing injectant and receiving plunger. A needle assembly is retractable into a retraction tube adjacent to the barrel. The barrel is in fluid communication with the needle via a lateral channel.

All of the prior syringes mentioned above require the user to fill the syringe by placing the needle in a vial or other container containing injectant fluid and drawing the injectant fluid into the syringe. Thus, the syringe is charged by the operator immediately prior to use. However, approximately 45% of injections are currently given using a pre-loaded syringe which is typically smaller in capacity than operator loaded syringes. It is expected that the use of pre-loaded syringes will increase significantly with time.

Thus, there is a need to provide a pre-loaded syringe with a retractable assembly.

### Summary of the invention

In accordance with the invention, there is provided a syringe including a needle retractable by stored energy on completion of an injection stroke, comprising a housing and a plunger, characterised in that a sealed capsule assembly containing the injectant is mounted in the housing and the sealed capsule assembly includes a needle assembly comprising a needle mounting, a needle, a frangible seal and a needle sheath covering the needle, wherein the sealed needle sheath prevents movement of the plunger into the housing before the seal is broken and the sheath is removed.

Thus in accordance with the invention, a syringe is provided which may be pre-charged with injectant, and for which the needle is automatically withdrawn after use to avoid the risk of needle stick injury.

### Brief description of the drawings

In order that the invention may be more fully understood an embodiment therefore will now be described by way of example with reference to the accompanying drawings in which:
Figure 1 is a schematic cross sectional view of an embodiment of syringe in accordance with the invention prior to use;
Figure 2 is a schematic cross section of the syringe shown in Figure 1, after use, with the needle withdrawn into the barrel of the device;
Figure 3 is a schematic cross section of a first alternative embodiment of the invention;
Figure 4 is a schematic cross section of the second alternative embodiment according to the invention;
Figure 5A is a schematic perspective view of the capsule assembly, including the needle and needle sheath; and
Figure 5B is a schematic illustration of an alternative needle capsule assembly.

### Detailed description

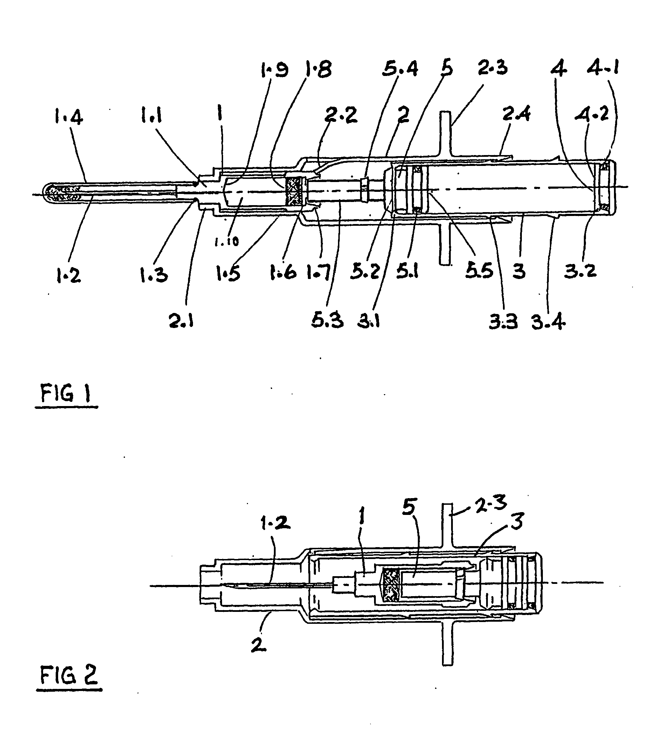

Figure 1 illustrates an embodiment of syringe in accordance with the invention which comprise a capsule assembly 1 which is pre-charged with injectant and includes the needle assembly with a needle sheath as will be described in more detail hereinafter. The capsule assembly is mounted in a cylindrical barrel 2 in which is mounted a plunger 3 provided with a closure piece 4 and a piston 5.

The capsule 1 containing injectant is integral with a needle mounting 1.1 and a needle 1.2. The needle mounting 1.1 contains a frangible sealant 1.3 to give a positive retention for a needle sheath 1.4.

The capsule assembly is of a diameter to fit through an opening 2.1 in the barrel 2 that has a region 1.5 of increased diameter in order to locate the capsule in place. The open end of the capsule 1 has an internal lip 1.6 and an external lip 1.7 and is provided with a resilient plunger disc 1.8. The needle end of the capsule 1.9 is dished. Injectant 1.10 is pre-charged into the capsule assembly 1 between the disc 1.8 and the dished end 1.9.

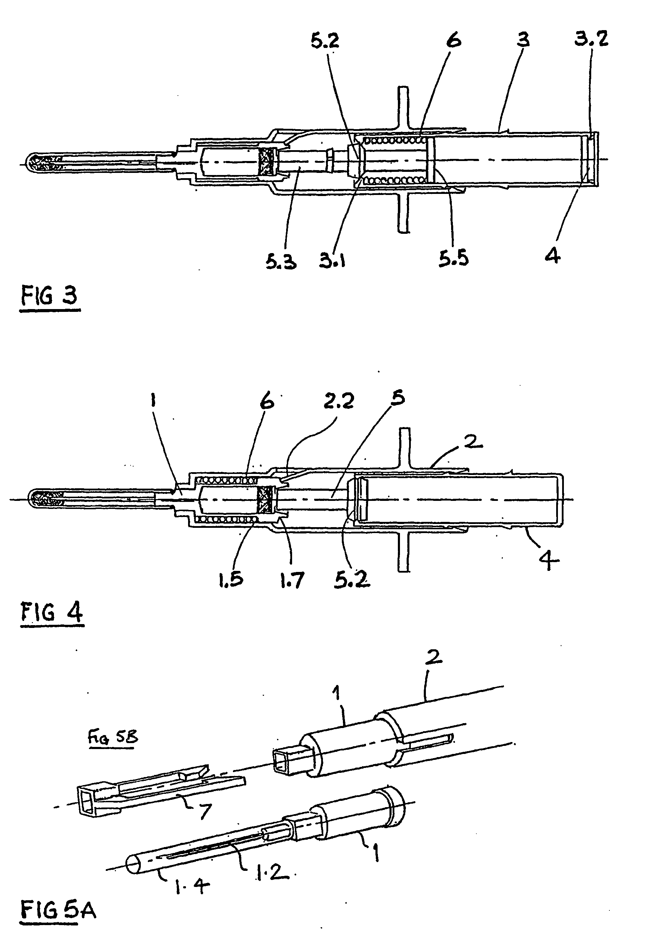

The barrel 2 has a nose 2.1, the aperture of which is larger than the diameter of the needle sheath 1.4. One or more arms 2.2, which may be part of the barrel 2, are positioned so that they abut against the external lip 1.7 of the capsule assembly. The barrel 2 has a finger plate 2.3 and an inwardly facing wedge sectioned ring 2.4. The nose 2.1 of the barrel and the needle mounting 1.1 may be non-circular as will be described hereinafter with reference to Figure 5, in order to prevent relative axial rotation.

The plunger 3 has an inwardly facing triangular sectioned ring 3.1, an inward facing wedge shaped ring 3.2 and outwardly facing wedge sectioned rings 3.3 and 3.4. The closure piece 4 has a seal 4.1 which is held in place by a wedge section ring 3.2 of plunger 3.

The piston 5 carries the seal 5.1 which co-acts with the inner surface of the plunger 3 and a triangular section ring 5.2. The piston 5 includes a probe 5.3 which carries a resilient slit ring 5.4. The ring 5.4 is positioned so that it snaps past internal lip 1.6 before the injection stroke is completed.

During assembly, the closure piece 4 and the piston 5 are temporarily held in contact by the use of an appropriate lubricant between the two mating surfaces 4.2 and 5.5. These are inserted as one piece at the right-hand end of the plunger 3 shown in Figure 1. The piston 5 is then drawn through the interior of the plunger 3 until the triangular sectioned ring 5.2 snaps past the inward facing triangular ring 3.1, creating a vacuum inside the plunger 3 and holding the piston 5 and the plunger 3 together as one piece. The vacuum is maintained by seals 4.1 and 5.1. The closure piece 4 is thus held at the end of the plunger 3.

Considering the capsule assembly 1, it is pre-charged with injectant fluid between the dished needle end 1.9 and the resilient plunger disc 1.8. Also, the assembly is fitted with the needle sheath 1.4 over the needle 1.2, which is held in place by the frangible sealant 1.3.

The capsule assembly 1 is passed through the interior of the barrel 2 until the needle mounting 1.1 rests in the nose 2.1 of the barrel. The aperture 2.1 is sufficiently large to allow the passage of the needle 1.2 and the protective, sealed sheath 1.4 through the aperture. The sheath 1.4 affords protection to the needle 1.2 and guidance in entering and locating the capsule assembly in the nose 2.1 of the barrel. The larger size of the nose aperture 2.1 in relation to the diameter of the needle 1.2 enables a bent needle to be retracted as will be evident hereinafter. The or each arm 2.2 abuts against the external lip 1.7 thereby restraining the capsule assembly 1 from inward movement. The seal afforded by the sheath 1.4 prevents inward movement of the plunger 3 being made before the sheath 1.4 has been removed from the needle 1.2.

The plunger 3 and the piston 5 are inserted into the barrel 2 as one piece until wedge sectioned ring 3.3 snaps past wedge sectioned ring 2.4. It is not possible thereafter for the plunger to be withdrawn from the barrel.

The syringe is thus assembled ready for use.

When it is desired to use the syringe, the sheath 1.4 is removed from the needle 1.2. The non-circular construction of the needle mounting 1.1 and the nose 2.1 allows the sheath 1.4 to be rotated independently of its associated components, thereby breaking the seal between them and facilitating removal of the sheath 1.4. The syringe can then be operated according to established practice for disposable syringes. Thus, the syringe is gripped by the user such that the plunger 3 is slidably inserted into the barrel 2, with the user gripping the finger plate 2.3. On completion of the injection stroke, automatic needle retraction is triggered by further pressure on the plunger 3, as will now be described in detail.

Pressure on the plunger 3 moves the plunger and the piston 5 together as a unit through the interior of the barrel 2 towards the capsule assembly 1. As the probe 5.3 enters the open end of the capsule 1, it forces the sealing disc 1.8 towards the needle end of the capsule thereby expelling the injectant 1.10 through the needle 1.2. As the disc 1.8 reaches the closed end 1.9 of the capsule, the resilient split ring 5.4 is compressed so as to move past the internal lip 1.6 and the plunger 3 lifts arm 2.2 away from the internal lip 1.7. Final inward movement of the probe 5.3 distorts the disc 1.8 against the dished end 1.9 of the capsule 1, thereby ensuring complete evacuation of the injectant. When the piston 5 reaches the end of the barrel 2, further inward movement of the piston 5 is prevented so that further inward pressure on the plunger 3 snaps the triangular ring 3.1 past the wedge sectioned ring 5.2, thereby releasing the piston 5 and allowing the vacuum within the plunger 3 to draw the piston 5 and the capsule assembly 1 into the plunger 3.

As this retraction commences, the pressure exerted by the probe 5.3 on the disc 1.8 is released. This allows the disc 1.8 to revert to its normal shape and in doing so, residual injectant in the needle 1.2 is drawn into the resultant cavities thereby preventing any seepage of residual injectant from the needle 1.2.

Towards the end of the injection stroke, and before retraction has commenced, lip 3.4 snaps past wedge section ring 2.4 thus locking the plunger 3 with the barrel 2 and preventing the plunger from being withdrawn therefrom.

Figure 2 illustrates the resulting configuration of the syringe component shown in Figure 1, after needle retraction has occurred. It will be seen that the needle 1.2 has been withdrawn completely within the barrel 2 thereby obviating the risk of needle stick injury.

Figure 3 illustrates an alternative construction in which the energy used to effect retraction is provided by compression spring 6 rather than the vacuum between the plunger 3 and piston 5, of the embodiment of Figure 1. In the embodiment of Figure 3, the spring 6 is located within the plunger 3 and is of the maximum diameter allowed by the internal dimensions of the plunger 3. The triangular section ring 3.1 provides a stop which locates the spring 6. The probe 5.3 is passed through the interior of the spring 6 and inward pressure is applied to face 5.5, moving the piston 5 through the plunger 3 simultaneously compressing the spring 6 until the triangular sectioned ring 5.2 snaps past the triangular sectioned ring 3.1. The spring 6 is now held in compression and the plunger 3 and piston 5 are held together as one piece. The closure piece 4 is now fitted into the barrel and held by the wedge sectioned ring 3.2. Operation of the device is as previously described with reference to Figure 1, with the retraction of the needle being performed under the force of compression spring 6 rather than the vacuum as previously described.

Figure 4 illustrates a configuration in which the compression spring 6 is located within the barrel 2, so as to surround the capsule assembly 1. The capsule assembly 1 is passed through the interior of the spring 6 which is compressed against the locally increased diameter 1.5 as the capsule assembly 1 is loaded into place. The capsule assembly 1 is held in position and the spring 6 is held in compression by the or each arm 2.2. acting on the external lip 1.7. The piston 5 has a triangularly shaped groove 5.2. In the embodiment of Figure 4, assembly is achieved as previously described, except that the piston 5 and the plunger 3 are joined by pushing the piston 5 into the plunger 3 until the triangularly shaped groove 5.2 is retained by the triangular section ring 3.1. Operation of the embodiment of Figure 4 is carried out as previously described except that when the final inward pressure on the plunger 3 snaps the triangular section ring 3.1 out of the groove 5.2, the spring 6 forces the capsule assembly 1 and the piston 5 into the plunger 3.

Figure 5A illustrates in perspective the capsule assembly 1 including the needle 1.2 and needle sheath 1.4 with its rectangular i.e. non-cylindrical nose portion 1.1 which co-acts with the close fitting, similarly shaped aperture in the barrel 2 (not shown in Figure 5). Thus, rotation of the needle and capsule assembly 1 is prevented by the co-acting non-circular faces of the assembly 1 and barrel aperture 2.1 when the needle sheath is twisted so as to break the seal 1.3 and facilitate removal of the sheath. It will be understood that other means of achieving this may be used such as an off-set pin and socket or other means of producing non-rotary engagement between the capsule assembly 1 and the barrel 2.

Figure 5B illustrates an alternative configuration in which the capsule assembly 1 can be retained in the housing 2 before retraction of the needle is initiated, using a clip 7.

The described embodiments of the invention thus provide for safe and reliable operation of a pre-loaded syringe and have the advantage that the capsule assembly 1 containing the injectant 1.10 is pre-loaded and sealed under controlled conditions ready for incorporation into the syringe. Furthermore the injectant and the quantity thereof may be identified on the capsule, for example by means of a barcode which may be magnetic and may provide data for inclusion into the patients record, stock control and quality control purposes. Additionally, the user is not required to expel excess air from the needle prior to use and no calibrations for the dose are necessary because the dose is pre-set by the volume of injectant included into the capsule assembly 1. It will be understood that partial rotation of the needle sheath 1.4 breaks the seal and facilitates removal of the needle sheath from the needle 1.4 immediately prior to use. Seepage and spillage of the injectant are prevented as previously described.

Thus, the syringe according to the invention is reliable, instinctive in its operation and capable of being used with one hand. Furthermore, the needle is automatically retracted completely following the injection and, because the aperture 2.1 in the nose of barrel is relatively large, it has the capacity to retract a bent needle. Furthermore, re-exposure of the needle cannot occur after the injection thereby minimising the risk of a needle stick injury. Also, accidental retraction of the needle before the injection is prevented.

The described examples of syringe according to the invention are so configured that there are locked closed after use to provide for compact and safe disposal. The described syringes can be manufactured at low cost.

## Claims

1. A syringe including a needle (1.2) retractable by stored energy on completion of an injection stroke, comprising a housing (2) and a plunger (3), **characterised in that** a sealed capsule assembly (1) containing the injectant is mounted in the housing (2) and the sealed capsule assembly (1) includes a needle assembly comprising a needle mounting (1.1), a needle (1.2), a frangible seal (1.3) and a needle sheath (1.4) covering the needle (1.2), wherein the sealed needle sheath (1.4) prevents movement of the plunger (3) into the housing (2) before the seal (1.3) is broken and the sheath (1.4) is removed.

2. A syringe as claimed in claim 1 in which only the needle and capsule assembly (1) contact the injectant.

3. A syringe as claimed in claim 1 or claim 2 in which the needle sheath (1.4) is provided with coding indicating injectant constituents and quantity.

4. A syringe as claimed in claim 3 in which the coding is by bar code.

5. A syringe as claimed in claim 3 in which the coding is magnetic.

6. A syringe as claimed in claim 3 in which the coding provides data for patients' records, stock control and quality control purposes.

7. A syringe as claimed in any preceding claim in which the stored energy is provided by a vacuum.

8. A syringe as claimed in claims 1 to 6 in which the stored energy is contained in a spring (6).

9. A syringe as claimed in claim 8, wherein the spring (6) is maintained in compression and located within the plunger (3).

10. A syringe as claimed in claim 8 in which the spring (6) is maintained in compression and located around the capsule assembly (1).

11. A syringe as claimed in claims 7 and 9 in which the retractable part of the plunger (3) couples to the capsule (1) and needle assembly near completion of the injection stroke.

12. A syringe as claimed in any preceding claim in which the plunger (3) is axially collapsible at a set overload.

13. A syringe as claimed in any preceding claim in which the needle and capsule assembly (1) is retained prior to retraction by a catch (2.2) displaceable by the plunger (3) on completion of the injection stroke.

14. A syringe as claimed in claim 13 in which the said catch (2.2) is an integrally formed feature of the housing (2).

15. A syringe as claimed in claim 13 in which the said catch is a separate component or assembly.

16. A syringe as claimed in any preceding claim in which the needle and capsule assembly (1) can be installed through the housing (2) of the syringe.

17. A syringe as claimed in any preceding claim in which the needle end of the housing (2) is provided with a non-cylindrical aperture through which the needle (1.2) and the sheath (1.4) can pass and into which a co-acting non-cylindrical section of the capsule assembly (1) enters to allow a torque to be applied to the sheath (1.4) to break the seal (1.3) and facilitate its removal.

18. A syringe as claimed in claim 17 in which the clearance provided by the non-cylindrical aperture in the housing (2) allows the stored energy to retract and retain within the housing (2) a bent needle (1.2).

19. A syringe as claimed in claims 1 to 16 in which the needle end of the housing (2) is provided with a cylindrical aperture through which the needle (1.2) and the sheath (1.4) can pass and into which a co-acting cylindrical section of the capsule assembly (1) enters, relative rotational movement being prevented by co-acting protuberance and receptor on the two parts, to allow a torque to be applied to the sheath (1.4) to break the seal (1.3) and facilitate its removal.

20. A syringe as claimed in claim 19, in which the clearance by the cylindrical aperture in the housing (2) allows the stored energy to retract and retain within the housing (2) a bent needle (1.2).

21. A syringe as claimed in any preceding claim in which the needle and capsule assembly (1) contains an axially moveable resilient disc (1.8) which under inward pressure will expel the injectant.

22. A syringe as claimed in claim 21 in which the resilience of the disc (1.8) allows distortion of its natural shape under pressure of the injection stroke to replicate the geometry of the needle end of the capsule (1) to provide complete evacuation of the capsule (1).

23. A syringe as claimed in claim 21 in which the resilience of the disc (1.8) allows return to its natural shape after the pressure of the injection stroke is released and thereby drawing in any residual injectant from the needle bore.

## Patentansprüche

1. Spritze, die eine Nadel (1.2) umfasst, die nach einer Injektion mittels gespeicherter Energie eingezogen werden kann, und ein Gehäuse (2) und einen Kolben (3) aufweist, **dadurch gekennzeichnet, dass** eine dicht verschlossene Kapseleinheit (1), die das Injektionsmittel enthält, in dem Gehäuse (2) eingebracht ist, und die dicht verschlossene Kapseleinheit eine Nadelanordnung aufweist, die eine Nadelbefestigung (1.1), eine Nadel (1.2), ein zerbrechbares Siegel (1.3) und eine die Nadel umhüllende Nadelkappe (1.4) umfasst, wobei die abgedichtete Nadelkappe (1.4) verhindert, dass sich der Kolben (3) vor dem Aufbrechen des Siegels (1.3) und Entfernen der Kappe (1.4) in das Gehäuse (2) bewegt.

2. Nadel nach Anspruch 1, bei der nur die Nadel und die Kapseleinheit (1) mit dem Injektionsmittel in Kontakt stehen.

3. Nadel nach Anspruch 1 oder 2, bei der die Nadelkappe (1.4) mit einer Kodierung versehen ist, die Bestandteile und die Menge des Injektionsmittels angibt.

4. Nadel nach Anspruch 3, bei der zur Kodierung ein Barcode verwendet wird.

5. Nadel nach Anspruch 3, bei der die Kodierung magnetisch ist.

6. Nadel nach Anspruch 3, bei der die Kodierung Daten für Patientenakten, Lagerhaltung und für Zwecke der Qualitätskontrolle bereitstellt.

7. Nadel nach einem der vorhergehenden Ansprüche, bei der die gespeicherte Energie in Form eines Vakuums vorliegt.

8. Nadel nach einem der Ansprüche 1 bis 6, bei der die gespeicherte Energie in einer Feder (6) gespeichert ist.

9. Nadel nach Anspruch 8, bei der die Feder (6) in zusammengedrücktem Zustand gehalten wird und innerhalb des Kolbens (3) angeordnet ist.

10. Nadel nach Anspruch 8, bei der die Feder (6) in zusammengedrücktem Zustand gehalten wird und um die Kapseleinheit (1) herum angeordnet ist.

11. Nadel nach Anspruch 7 und 9, bei der der zurückziehbare Teil des Kolbens (3) gegen Ende der Injektion an die Kapsel (1) und die Nadelanordnung anschließt.

12. Nadel nach einem der vorhergehenden Ansprüche, bei der der Kolben (3) bei einer festgelegten Überlast axial stauchbar ist.

13. Nadel nach einem der vorhergehenden Ansprüche, bei der Nadelanordnung und Kapseleinheit (1) vor dem Zurückziehen an einem Anschlag (2,2) gehalten werden, der bei Beendigung der Injektion durch den Kolben (3) verdrängt werden kann.

14. Nadel nach Anspruch 13, bei der der Anschlag (2.2) eine mit dem Gehäuse (2) einstückig ausgeführte Einrichtung ist.

15. Nadel nach Anspruch 13, bei der der Anschlag eine eigenständige Komponente oder Einheit ist.

16. Nadel nach einem der vorhergehenden Ansprüche, wobei Nadelanordnung und Kapseleinheit durch das Gehäuse (2) der Spritze eingebracht werden können.

17. Nadel nach einem der vorhergehenden Ansprüche, bei der das Nadelende des Gehäuses (2) mit einer nicht-zylindrischen Öffnung ausgestattet ist, durch die die Nadel (1.2) und die Nadelkappe (1.4) hindurchtreten können und in die ein zusammenwirkender nicht-zylindrischer Abschnitt der Kapseleinheit (1) eingebracht wird, um zu ermöglichen, dass auf die Kappe (1.4) ein Drehmoment ausgeübt werden kann, um das Siegel (1.3) zu zerbrechen und deren Entfernen zu erleichtern.

18. Nadel nach Anspruch 17, bei der der Zwischenraum, der durch die nicht-zylindrische Öffnung in dem Gehäuse (2) geschaffen wird, es ermöglicht, dass eine gebogene Nadel (1.2) durch die gespeicherte Energie zurückgezogen und in dem Gehäuse (2) aufgenommen werden kann.

19. Nadel nach einem der Ansprüche 1 bis 16, bei der das Nadelende des Gehäuses (2) mit einer zylindrischen Öffnung ausgestattet ist, durch die die Nadel (1.2) und die Nadelkappe (1.4) hindurchtreten können und in die ein zusammenwirkender zylindrischer Abschnitt der Kapseleinheit (1) eingebracht wird, wobei eine relative Drehbewegung durch einen Vorsprung und eine Aufnahme an den beiden Teilen, die zusammenwirken, verhindert wird, um zu ermöglichen, dass auf die Kappe (1.4) ein Drehmoment ausgeübt werden kann, um das Siegel (1.3) zu zerbrechen und Vereinfachen deren Entfernen zu erleichtern.

20. Nadel nach Anspruch 19, bei der der durch die zylindrische Öffnung in dem Gehäuse (2) geschaffene Zwischenraum es ermöglicht, dass eine gebogene Nadel (1.2) durch die gespeicherte Energie zurückgezogen und in dem Gehäuse (2) aufgenommen werden kann.

21. Nadel nach einem der vorhergehenden Ansprüche, bei der Nadelanordnung und Kapseleinheit (2) eine axial bewegliche elastische Scheibe (1.8) aufweisen, die durch einen nach innen gerichteten Druck das Injektionsmittel ausstößt.

22. Nadel nach Anspruch 21, bei der die Elastizität der Scheibe (1.8) eine Verformung von deren ursprünglicher Form unter dem Druck der Injektion ermöglicht, sodass sie für eine vollständige Entleerung der Kapsel (1) die Geometrie des Nadelendes der Kapsel (1) annimmt.

23. Nadel nach Anspruch 21, bei der die Scheibe (1.8) durch ihre Elastizität nach dem Nachlassen des Drucks der Injektion in ihre ursprüngliche Form zurückkehrt und dadurch das gesamte restliche Injektionsmittel aus der Nadelbohrung gezogen wird.

## Revendications

1. Seringue incluant une aiguille (1.2) rétractable par l'énergie stockée lors de l'achèvement d'une course d'injection, comprenant un boîtier (2) et un plongeur (3), **caractérisée en ce qu'**un ensemble de capsule scellé (1) contenant l'injectant est installé dans le boîtier (2), et l'ensemble de capsule scellé (1) comprend un ensemble d'aiguille comprenant un montage d'aiguille (1.1), une aiguille (1.2), un scellement pouvant être rompu (1.3) et une gaine de protection d'aiguille (1.4) couvrant l'aiguille (1.2), où la gaine de protection d'aiguille scellée (1.4) empêche un mouvement du plongeur (3) dans le boîtier (2) avant la rupture du scellement (1.3) et du retrait de la gaine de protection (1.4).

2. Seringue selon la revendication 1, dans laquelle seulement l'aiguille et l'ensemble de capsule (1) viennent en contact avec l'injectant.

3. Seringue selon la revendication 1 ou la revendication 2, dans laquelle la gaine de protection d'aiguille (1.4) est munie d'un codage indiquant les constituants de l'injectant ainsi que la quantité.

4. Seringue selon la revendication 3, dans laquelle le codage est un code à barres.

5. Seringue selon la revendication 3, dans laquelle le codage est magnétique.

6. Seringue selon la revendication 3, dans laquelle le codage fournit des données pour les dossiers des patient, pour la gestion des stocks et pour le contrôle de qualité.

7. Seringue selon l'une quelconque des revendications précédentes, dans laquelle l'énergie stockée est réalisée par un vide.

8. Seringue selon les revendications 1 à 6, dans laquelle l'énergie stockée est retenue dans un ressort (6).

9. Seringue selon la revendication 8, dans laquelle le ressort (6) est maintenu en compression et est situé dans le plongeur (3).

10. Seringue selon la revendication 8, dans laquelle le ressort (6) est maintenu en compression et est situé autour de l'ensemble de capsule (1).

11. Seringue selon les revendications 7 et 9, dans laquelle la partie rétractable du plongeur (3) est couplée à la capsule (1) et à l'ensemble d'aiguille proche de l'achèvement de la course d'injection.

12. Seringue selon l'une quelconque des revendications précédentes, dans laquelle le plongeur (3) est apte à s'affaisser axialement à une surcharge réglée.

13. Seringue selon l'une quelconque des revendications précédentes, dans laquelle l'ensemble d'aiguille et de capsule (1) est retenu avant la rétraction par un cliquet (2.2) déplaçable par le plongeur (3) lors de l'achèvement de la course d'injection.

14. Seringue selon la revendication 13, dans laquelle ledit cliquet (2.2) est une propriété réalisée intégralement du boîtier (2).

15. Seringue selon la revendication 13, dans laquelle ledit cliquet est un composant ou ensemble séparé.

16. Seringue selon l'une quelconque des revendications précédentes, dans laquelle l'ensemble d'aiguille et de capsule (1) peut être installé à travers le boîtier (2) de la seringue.

17. Seringue selon l'une quelconque des revendications précédentes, dans laquelle l'extrémité d'aiguille du boîtier (2) est munie d'une ouverture non cylindrique à travers laquelle l'aiguille (1.2) et la gaine de protection (1.4) peuvent passer dans laquelle une section non cylindrique de coaction de l'ensemble de capsule (1) entre pour permettre l'application d'un couple à la gaine de protection (1.4) pour rompre le scellement (1.3) et pour faciliter son retrait.

18. Seringue selon la revendication 17, dans laquelle le jeu réalisé par l'ouverture non cylindrique dans le boîtier (2) permet à l'énergie stockée de rétracter et retenir dans le boîtier (2) une aiguille courbée (1.2).

19. Seringue selon les revendications 1 à 16, dans laquelle l'extrémité d'aiguille du boîtier (2) est munie d'une ouverture cylindrique à travers laquelle l'aiguille (1.2) et la gaine de protection (1.4) peuvent passer et dans laquelle une section cylindrique de coaction de l'ensemble de capsule (1) entre, un mouvement de rotation relatif étant empêché par une saillie et un logement récepteur en coaction sur les deux parties, pour permettre l'application d'un couple à la gaine de protection (1.4) pour rompre le scellement (1.3) et pour faciliter son retrait.

20. Seringue selon la revendication 19, dans laquelle le jeu réalisé par l'ouverture cylindrique dans le boîtier (2) permet à l'énergie stockée de rétracter et retenir dans le boîtier (2) une aiguille courbée (1.2).

21. Seringue selon l'une quelconque des revendications précédentes, dans laquelle l'ensemble à aiguille et à capsule (1) contient un disque résilient déplaçable axialement (1.8) qui, sous pression exercée vers l'intérieur, expulsera l'injectant.

22. Seringue selon la revendication 21, dans laquelle la résilience du disque (1.8) permet la distorsion de sa forme naturelle sous pression de la course d'injection de répliquer la géométrie de l'aiguille et de la capsule (1) pour obtenir une évacuation complète de la capsule (1).

23. Seringue selon la revendication 21, dans laquelle la résilience du disque (1.8) permet le retour à sa forme naturelle après le relâchement de la pression de la course d'injection et de ce fait l'aspiration de tout injectant résiduel du perçage d'aiguille.
